# EUROPEAN PATENT SPECIFICATION

(11) **EP 1 598 085 B1**
(45) Date of publication and mention of the grant of the patent: **04.11.2009**
(21) Application number: 04708441.3
(22) Date of filing: 05.02.2004
(51) Int. Cl.: A61L 24/04

(54) **BIODEGRADABLE AND PRESSURE-SENSITIVE MATERIAL FOR MEDICAL USE**
BIOLOGISCH ABBAUBARES UND DRUCKEMPFINDLICHES MATERIAL FÜR MEDIZINISCHE ZWECKE
MATIERE BIODEGRADABLE ET SENSIBLE A LA PRESSION DESTINEE A UN USAGE MEDICAL

(30) Priority: 13.02.2003 JP 2003035710
(43) Date of publication of application: 23.11.2005
(73) Proprietor: National Institute for Materials Science, Tsukuba-shi, Ibaraki 305-0047 (JP); Furuuchi Chemical Corporation, Tokyo 140-0013 (JP)
(72) Inventor: TAGUCHI, Tetsushi, Nat.Inst. For Materials Science, Tsukuba-shi, Ibaraki 305-0047 (JP); KOBAYASHI, Hisatoshi, Nat.Inst. Materials Science, Tsukuba-shi, Ibaraki 305-0047 (JP); TANAKA,Junzo, Nat.Inst. For Materials Science, Tsukuba-shi, Ibaraki 305-0047 (JP); SAITO, Hirofumi, Furuuchi Chemical Corporation, Tokyo 140-0013 (JP)
(74) Representative: Röthinger, Rainer
(86) International application number: PCT/JP2004/001194
(87) International publication number: WO 2004/071544

(56) References cited:
- JP-A- 6 296 679
- JP-A- 9 103 479
- JP-A- 9 296 039
- KR-A- 2001 096 018
- US-A- 4 621 631
- US-A- 5 804 428
- PATENT ABSTRACTS OF JAPAN vol. 1998, no. 03, 27 February 1998 (1998-02-27) -& JP 09 296039 A (EIWEISS KK), 18 November 1997 (1997-11-18)
- PATENT ABSTRACTS OF JAPAN vol. 2000, no. 13, 5 February 2001 (2001-02-05) & JP 2000 290633 A (TOYOBO CO LTD), 17 October 2000 (2000-10-17)
- TAGUCHI ET AL: "Biocompatible high-strength glue consisting of citric acid derivative and collagen" MATERIALS SCIENCE AND ENGINEERING C, ELSEVIER SCIENCE S.A, CH, vol. 26, no. 1, January 2006 (2006-01), pages 9-13, XP005220795 ISSN: 0928-4931
- TAGUCHI T ET AL: "Bonding of soft tissues using a novel tissue adhesive consisting of a citric acid derivative and collagen" MATERIALS SCIENCE AND ENGINEERING C, ELSEVIER SCIENCE S.A, CH, vol. 24, no. 6-8, 1 December 2004 (2004-12-01), pages 775-780, XP004637947 ISSN: 0928-4931
- IWATA, MATSUDA, MITSUHASHI,ITOH IKADA: "A novel surgical glue composed of gelatin and N-hydroxysuccinimide activated poly(L-glutamic acid)" BIOMATERIALS, vol. 19, 1998, pages 1869-1976,

## Description

### TECHNICAL FIELD

The present invention relates to a novel two-component, bio-degradable/absorbable adhesive medical material comprising a biodegradable polymer serving as a bonding component and a biological low-molecular-weight derivative serving as a hardening component.

### BACKGROUND ART

The occlusion/joining of a wound site in the skin, internal organ, blood vessel or the like is one of the most fundamental techniques for use in surgical operations or the like, and generally performed by sutures using a thread in this day and age. On the other hand, a technique for quickly occluding/joining a wound site by means of a biologically compatible adhesive having an appropriate tension resistance without using the suture thread has been developed, and biological tissue adhesives, such as fibrin-based adhesives, cyanoacrylate-based adhesives and polyurethane-based adhesives, have been clinically used therein. As another medical material, there has also been known a biological tissue adhesive having a bonding component comprising a biodegradable polymer, such as gelatin or collagen (see, for example, the following Patent Publications 1 to 6).
Patent Publication 1: Japanese Patent Laid-Open Publication No. 06-218035
Patent Publication 2: Japanese Patent Laid-Open Publication No. 07-163860
Patent Publication 3: Japanese Patent Laid-Open Publication No. 09-103479
Patent Publication 4: WO 98/54224
Patent Publication 5: Japanese Patent Laid-Open Publication No. 2000-290633
Patent Publication 6: Japanese Patent Laid-Open Publication No. 2000-503883 (Patent No. 3238711)

Further, US 4,621,631 describes the production of a bonded collagen fiber sheet suitable as covering for wounds by isolating collagen fibers from collagen containing material, preferably human placenta, crosslinking the isolated collagen fibers using a crosslinking agent, preferably formaldehyde, and forming sheets from the obtained bonded collagen fibers. In addition, JP 09 296039 A discloses a tissue adhesive comprising N-hydroxysuccinimide activated poly(L-glutamic acid) (NHS-PLGA) and gelatin.

### DISCLOSURE OF INVENTION

### (Problem to be solved by the Invention)

Heretofore, there has not been any biological tissue adhesive having strong tissue- bonding force, and low biological toxicity in a remaining crosslinking agent or a degradation product. Moreover, a conventional hemostatic material, blood-vessel embolizing material, sealant or aneurysm closing material has been liable to peel off from its applied site and has not been able to achieve sufficient hemostatic effect or sealing/closing strength in occluding a blood vessel, stopping bleeding, sealing air-leak or closing an aneurysm.

### (Means for solving the Problem)

In view of the above problems, the present invention is directed to a development of a bio-degradable/absorbable adhesive medical material having high bonding strength between tissues and low biological toxicity, through the use of a hardening component comprising a low-molecular-weight derivative derived from a biological molecule.

Specifically, the present invention provides a two-component, bio-degradable/absorbable adhesive medical material comprising a bonding component which consists of a solution containing a biodegradable polymer and either one of an organic solvent, water and a mixture of water and an organic solvent, and a hardening component which consists of a low-molecular-weight derivative prepared by modifying at least two carboxyl groups in one of malic acid, oxalacetic acid, citric acid, cis-aconitic acid, and 2-ketoglutaric acid with an electron-attracting group.

In this medical material, the electron-withdrawing group may be one or a combination of two or more selected from the group consisting of a succinimidyl group, a sulfosuccinimidyl group, a maleimidyl group, a phthalimidyl group, an imidazolyl group, a nitrophenyl group and a tresyl group, and derivatives thereof.

The biodegradable polymer to be used in the bonding component may be a natural biodegradable polymer which is one or a combination of two or more selected from the group consisting of collagen, atelocollagen, alkali-solubilized collagen, gelatin, keratin, albumin, globulin, fibrinogen, glycosaminoglycan, chitin and chitosan, or may be a synthetic biodegradable polymer which is one or a combination of two or more selected from the group consisting of polyamino acid and polyalcohol.

The solvent for dissolving the biodegradable polymer may be one or a combination of two or more selected from the group consisting of distilled water, buffer solution and organic solvent.

Among them, the organic solvent may be one or a combination of two or more selected from the group consisting of dimethyl sulfoxide (DMSO), N,N-dimethylformamide (DMF), lactic acid, lactic acid oligomer, polyethylene glycol and polypropylene glycol.

### BRIEF DESCRIPTION OF DRAWINGS

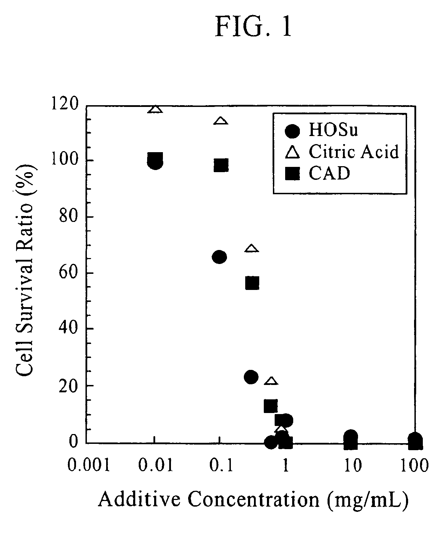

FIG. 1 is a graph showing the result of a cytotoxicity test on three compounds related to the synthesis of a citric acid derivative (CAD).

### BEST MODE FOR CARRYING OUT THE INVENTION

A biological low-molecular-weight derivative to be used in the present invention is prepared by introducing an active ester into a di- or tri-carboxylic acid of the citric acid cycle selected from the group consisting of malic acid, oxalacetic acid, citric acid, cis-aconitic acid, 2-ketoglutaric acid through the reaction between the di- or tri-carboxylic acid, and an electron-attracting group, such as one or a combination of two or more selected from the group consisting of a succinimidyl group, a sulfosuccinimidyl group, a maleimidyl group, a phthalimidyl group, an imidazolyl group, a nitrophenyl group and a tresyl group.

More specifically, the biological low-molecular-weight derivative in the present invention may be obtained by adding a molecule serving as an electron-attracting group, such as N-hydroxysuccinimide, to an organic solvent solution of the di- or tri-carboxylic acid of the citric acid cycle, under the presence of a condensing agent, such as 1-ethyl-3-(3-dimethylaminopropyl)carbodiimide, and purifying an obtained product by silica-gel column chromatography.

A biodegradable polymer to be used in the present invention may be a natural biodegradable polymer, such as one or a combination of two or more selected from the group consisting of collagen (which may be either one of several ten types), atelocollagen (which may be either one of several ten types), alkali-solubilized collagen (which may be either one of several ten types), gelatin, keratin, albumin, globulin, fibrinogen, glycosaminoglycan, chitin and chitosan (which may have any degree of deacetylation or any molecular weight). The protein may be derived from any suitable source organism. The biodegradable polymer to be used in the present invention may also be a synthetic biodegradable polymer, such as one or a combination of two or more selected from the group consisting of polyamino acid (which may be any type of amino acid or may have any molecular weight) and polyalcohol (which may be any type or may have any molecular weight), each of which has a functional group reactable with a biological low-molecular-weight molecule modified with an electron-withdrawing group.

The glycosaminoglycan may be one or a combination of two or more selected from the group consisting of chondroitin sulfate, dermatan sulfate, hyaluronic acid, heparan sulfate, heparin and keratan sulfate. Each of the glycosaminoglycans may have any molecular weight or may be derived from any suitable source organism.

As to the ratio between the bonding component (biodegradable polymer) and the hardening component (biological low-molecular-weight derivative), the hardening component may be set at 0.01 to 1000 mM with respect to 0.01 to 80 weight% of the bonding component in the solvent, such as organic solvent, distilled water or buffer solution. More preferably, the bonding component in the solvent is set in the range of 10 to 60 weight%. Further, the hardening component is set more preferably in the range of 10 to 200 mM with respect to the bonding component. The reaction between the bonding and hardening components is induced preferably at 0 to 100°C, more preferably at 4 to 60°C. Preferably, each of the bonding and hardening components is prepared as a solution having an appropriate concentration, and then they are mixed together to induce a homogenous reaction therebetween.

The solvent for forming the bonding-component solution or the hardening-component solution may include a buffer solution, such as physiological salt solution, sodium hydrogen carbonate, boric acid and phosphoric acid, as well as distilled water. The use of the buffer solution makes it possible to prevent a tissue around a site with the adhesive attached thereto from becoming necrotic due to osmotic pressure and/or change in pH.

The solvent may also be an organic solvent, such as one or a combination of two or more selected from the group consisting of dimethyl sulfoxide (DMSO), N,N-dimethylformamide (DMF), lactic acid, lactic acid oligomer and polyethylene glycol. Further, a mixed solvent prepared by mixing distilled water and organic solvent at an appropriate ratio may be used.

The above two component, bio-degradable/absorbable adhesive medical material may be used as a biological tissue adhesive for bonding between soft tissues, such as skin tissue-to-skin tissue, bonding between hard tissues, such as bone tissue-to-bone tissue, and bonding between hard and soft tissues, such as bone tissue-to-cartilage tissue. This medical material may also be used as a hemostatic material, a blood-vessel embolizing material, a sealant or an aneurysm closing material. When used in these purpose, the bio-degradable/ absorbable adhesive medical material of the present invention exhibits characteristics of being degraded in the body and then absorbed/vanished after a lapse of a given time-period, and thereby will never remain in the body as a foreign material.

### [EXAMPLE]

The present invention will be described in more detail below in connection with examples.

### CYTOTOXICITY TEST

A cytotoxicity test was carried out in the following manner. As one example of the low-molecular-weight derivative serving as the hardening component, a biological low-molecular-weight derivative (CAD) was synthesized by modifying three carboxyl groups of a citric acid with N-hydroxysuccinimide (HOSu), and a cytotoxicity test was performed using L929 cells and the synthesized CAD. The number of cells in a sample added with no CAD was defined as 100%. The CAD exhibited extremely low toxicity as with citric acid and HOSu.

### (Inventive Examples 1 to 3)

Alkali-solubilized collagen (AlCol) was used as the biodegradable polymer. 50 µL of CAD solution containing a commercially available CAD serving as the hardening component dissolved therein in each amount of 100 mM (Inventive Example 1), 50 mM (Inventive Example 2) and 30 mM (Inventive Example 3), was added to 200 µL of DMSO solution containing 30 weight% of AlCol. The mixture was stirred at 25°C for several second to prepare a mixed solution.

### (Inventive Example 4)

50 µL of CAD solution containing 100 mM of commercially available CAD dissolved therein was added to 200 µL of DMSO solution containing 50 weight% of alkali-solubilized collagen (AlCol). The mixture was stirred at 25°C for several second to prepare a mixed solution.

A bonding test on Inventive Examples 1 to 4 was carried out using a commercially available ham. A tissue bonding test was performed in the following manner. Each of the prepared mixed solutions was applied onto a prepared ham (thickness: 2 mm, width: 2 cm, length: 6 cm) in an area of 2 × 2 cm², and a piece of ham having the same size was superimposed on the bonding surface. Further, 50 g of weight was loaded on the bonding surface, and the hams were left at 37°C for 12 hours. A bonding strength was measured by a tension tester (TA-XT 2i, available from Eko Instruments Co., Ltd., Japan). The measurement was performed at 25°C and a measurement speed of 5 mm/s. The test result is shown in Table 1.

**Table 1**

| | Bonding Conditions | Bonding Strength (kPa) |
|---|---|---|
| Inventive Example 1 | 30 wt% of AICol + CAD 100 mM | 13.1 |
| Inventive Example 2 | 30 wt% of AlCol + CAD 50 mM | 6.2 |
| Inventive Example 3 | 30 wt% of AICol + CAD 30 mM | 3.3 |
| Inventive Example 4 | 50 wt% of AlCol + CAD 100 mM | 19.9 |

From the comparison between Inventive Examples 1 to 3 in Table 1, it is proven that a higher CAD concentration provides a higher bonding strength. Further, from the comparison between Inventive Examples 1 and 4, it is proven that a higher AlCol concentration provides a higher bonding strength under the same CAD concentration.

### (Comparative Example 1)

A and B liquids of fibrin paste (Beriplast P®, available from Hoechst AG, Germany) were mixed to prepare a biological tissue adhesive. 0.20 g of this biological tissue adhesive was applied onto a prepared ham (thickness: 2 mm, width: 2 cm, length: 6 cm) in an area of 2 × 2 cm², and a piece of ham having the same size was superimposed on the bonding surface. Further, 50 g of weight was loaded on the bonding surface, and the hams were left at 37°C for 12 hours.

### (Comparative Example 2)

Gelatin paste (GRF-glue®, available from E. H. S., France) consisting of two liquids of a gelatin-resorcinol solution and a formaldehyde-glutaraldehyde solution was used as the biological tissue adhesive. 0.20 g of the gelatin-resorcinol solution was applied onto a prepared ham (thickness: 2 mm, width: 2 cm, length: 6 cm) in an area of 2 × 2 cm², and a piece of ham having the same size was superimposed on the bonding surface. Further, 50 g of weight was loaded on the bonding surface, and the hams were left at 37°C for 12 hours.

### (Comparative Example 3)

2-octyl cyanoacrylate (DERMABOND®, available from ETHICON. Inc., U.S.A.) was used. 0.25 g of this biological tissue adhesive was applied onto a prepared ham (thickness: 2 mm, width: 2 cm, length: 6 cm) in an area of 2 × 2 cm², and a piece of ham having the same size was superimposed on the bonding surface. Further, 50 g of weight was loaded on the bonding surface, and the hams were left at 37°C for 12 hours.

A bonding strength of each biological tissue adhesive in Comparative Examples 1 to 3 was measured by a tension tester (TA-XT 2i, available from Eko Instruments Co., Ltd., Japan). The measurement was performed at 25°C and a measurement speed of 5 mm/s. The test result on Comparative Examples 1 to 3 and the test result on Inventive Example 4 having the best bonding condition (AlCol concentration: 50 wt%, CAD concentration: 100 mM) are shown in Table 2.

**Table 2**

| Adhesive | Bonding Strength (kPa) |
|---|---|
| Inventive Example 4 | 15.8 |
| Comparative Example 1 | 0.7 |
| Comparative Example 2 | 15.2 |
| Comparative Example 3 | 25.8 |

As seen in Table 2, while the biological tissue adhesive in Inventive Example 4 using CAD is inferior to Comparative Example 3, it has a higher bonding strength than those of the biological tissue adhesives in Comparative Examples 1 and 2.

### INDUSTRIAL APPLICABILITY

The present invention provides a low-toxic bio-degradable/absorbable adhesive medical material usable as a medical material to be crosslinked extemporarily on the site of medical procedures, such as a hemostatic material, a blood-vessel embolizing material, a sealant or an aneurysm closing material, as well as a biological tissue adhesive. This bio-degradable/absorbable adhesive medical material has no need for taking account of toxicity in use, and can be handled in a significantly simple manner.

## Claims

1. A two-component, bio-degradable/absorbable biological tissue adhesive for bonding between a soft tissue and a soft tissue, between a soft tissue and a hard tissue or between a hard tissue and a hard tissue comprising:
a bonding component consisting of a solution containing a biodegradable polymer and either one of an organic solvent, water and a mixture of water and an organic solvent; and
a hardening component consisting of a low-molecular-weight derivative obtainable by modifying at least two carboxyl groups in one of malic acid, oxalacetic acid, citric acid, cis-aconitic acid, and 2-ketoglutaric acid with an electron-withdrawing group.

2. The biological tissue adhesive as defined in claim 1, wherein said electron-attracting group is one or a combination of two or more selected from the group consisting of a succinimidyl group, a sulfosuccinimidyl group, a maleimidyl group, a phthalimidyl group, an imidazolyl group, a nitrophenyl group and a tresyl group.

3. The biological tissue adhesive as defined in claim 1, wherein said biodegradable polymer is one or a combination of two or more selected from the group consisting of collagen, atelocollagen, alkali-solubilized collagen, gelatin, keratin, albumin, globulin, fibrinogen, glycosaminoglycan, chitin, chitosan, polyamino acid and polyalcohol.

4. The biological tissue adhesive as defined in claim 1, wherein said organic solvent is one or a combination of two or more selected from the group consisting of dimethyl sulfoxide (DMSO), N,N-dimethylformamide (DMF), lactic acid, lactic acid oligomer, polyethylene glycol and polypropylene glycol.

5. An intravascular material consisting of either one of a hemostatic material, a blood-vessel embolizing material, a sealant and an aneurysm closing material, comprising the biological tissue adhesive as defined in either one of claims 1 to 4.

## Patentansprüche

1. Biologisch abbaubarer/absorbierbarer, biologischer Zweikomponenten-Gewebekleber zum Verkleben eines Weichgewebes mit einem Weichgewebe, eines Weichgewebes mit einem Hartgewebe oder eines Hartgewebes mit einem Hartgewebe, umfassend:
eine Klebekomponente, bestehend aus einer Lösung, die ein biologisch abbaubares Polymer und entweder ein organisches Lösungsmittel, Wasser oder eine Mischung von Wasser und einem organischen Lösungsmittel enthält; und
eine Härtungskomponente, bestehend aus einem Derivat mit geringem Molekulargewicht, erhältlich durch Modifizieren wenigstens zweier Carboxylgruppen in Äpfelsäure, Oxalessigsäure, Zitronensäure, cis-Aconitsäure oder 2-Ketoglutarsäure mit einer elektronenziehenden Gruppe.

2. Biologischer Gewebekleber wie in Anspruch 1 definiert, wobei die elektronenanziehende Gruppe eine oder eine Kombination von zwei oder mehreren Gruppen, ausgewählt aus der Gruppe bestehend aus einer Succinimidylgruppe, einer Sulfosuccinimidylgruppe, einer Maleimidylgruppe, einer Phthalimidylgruppe, einer Imidazolylgruppe, einer Nitrophenylgruppe und einer Tresylgruppe, ist.

3. Biologischer Gewebekleber wie in Anspruch 1 definiert, wobei das biologisch abbaubare Polymer eines oder eine Kombination von zwei oder mehrerer ausgewählt aus der Gruppe bestehend aus Kollagen, Atelokollagen, mit Alkali solubisiertem Kollagen, Gelatine, Keratin, Albumin, Globulin, Fibrinogen, Glykosaminoglykan, Chitin, Chitosan, Polyaminosäure und Polyalkohol ist.

4. Biologischer Gewebekleber wie in Anspruch 1 definiert, wobei das organische Lösungsmittel eines oder eine Kombination zweier oder mehrerer ausgewählt aus der Gruppe bestehend aus Dimethylsulfoxid (DMSO), N,N-Dimethylformamid (DMF), Milchsäure, Milchsäureoligomer, Polyethylenglykol und Polypropylenglykol ist.

5. Intravaskuläres Material, bestehend entweder aus einem blutstillenden Material, einem Blutgefäss embolisierenden Material, einem Versiegelungsmittel oder einem Aneurysma verschließenden Material, umfassend den biologischen Gewebekleber wie in einem der Ansprüche 1 bis 4 definiert.

## Revendications

1. Adhésif pour tissus biologiques biodégradable/absorbable à deux composants pour une liaison entre un tissu mou et un tissu mou, entre un tissu mou et un tissu dur ou entre un tissu dur et un tissu dur comprenant :
un composant de liaison consistant en une solution contenant un polymère biodégradable et l'un ou l'autre d'un solvant organique, d'eau et d'un mélange d'eau et d'un solvant organique ; et
un composant de durcissement consistant en un dérivé de faible poids moléculaire pouvant être obtenu par modification d'au moins deux groupes carboxyle dans l'un de l'acide malique, de l'acide oxalacétique, de l'acide citrique, de l'acide cis-aconitique, et de l'acide 2-cétoglutarique avec un groupe de retrait d'électrons.

2. Adhésif pour tissus biologiques selon la revendication 1, dans lequel ledit groupe d'attraction d'électrons est un groupe ou une combinaison de deux groupes ou plus choisi(s) parmi le groupe consistant en un groupe succinimidyle, un groupe sulfosuccinimidyle, un groupe maléimidyle, un groupe phtalimidyle, un groupe imidazolyle, un groupe nitrophényle et un groupe trésyle.

3. Adhésif pour tissus biologiques selon la revendication 1, dans lequel ledit polymère biodégradable est un polymère ou une combinaison de deux polymères ou plus choisi(s) parmi le groupe consistant en le collagène, l'atélocollagène, un collagène solubilisé par un alcalin, une gélatine, la kératine, l'albumine, une globuline, le fibrinogène, le glycosaminoglycane, la chitine, le chitosane, un acide polyamino et un polyalcool.

4. Adhésif pour tissus biologiques selon la revendication 1, dans lequel ledit solvant organique est un solvant ou une combinaison de deux solvants ou plus choisi(s) parmi le groupe consistant en le diméthylsulfoxyde (DMSO), le N,N-diméthylformamide (DMF), l'acide lactique, un oligomère de l'acide lactique, un polyéthylène glycol et un polypropylène glycol.

5. Matériau intravasculaire consistant en l'un ou l'autre d'un matériau hémostatique, d'un matériau d'embolisation de vaisseau sanguin, d'un colmatant et d'un matériau de fermeture d'anévrisme, comprenant l'adhésif pour tissus biologiques tel que défini dans l'une ou l'autre des revendications 1 à 4.
